# EUROPÄISCHE PATENTSCHRIFT

(11) **EP 0 606 657 B1**
(45) Veröffentlichungstag und Bekanntmachung des Hinweises auf die Patenterteilung: **30.10.1996**
(21) Anmeldenummer: 93121130.4
(22) Anmeldetag: 30.12.1993
(51) Int. Cl.: C07D 487/04, A61K 31/40

(54) **Ketorolac-Derivat mit wesentlich geringerer gastrointestinaler Reizung und Ulzeration**
Ketorolac derivative having considerably less gastrointestinal irritation and ulceration
Dérivé de kétorolac à activité d'irritation et d'ulcération gastrointestinale diminuée

(30) Priorität: 13.01.1993 DE 4300697
(43) Veröffentlichungstag der Anmeldung: 20.07.1994
(73) Patentinhaber: ROEMMERS S.A.I.C.F., Buenos Aires 1086 (AR)
(72) Erfinder: Monti, Carlos Ernesto Antonio, 1714 Ituzaingo, Buenos Aires (AR); Aldomá, Gustavo Enrique, 1426 Buenos Aires (AR)
(74) Vertreter: Wibbelmann, Jobst, Dr., Dipl.-Chem.

(56) Entgegenhaltungen:
- EP-A- 0 289 262
- WO-A-91/13609
- DE-A- 2 731 678
- JOURNAL OF MEDICINAL CHEMISTRY, Bd.28, 1985 Seiten 1037 - 1049 J.M. MUCHOWSKI ET AL.
- PHARMACEUTICAL RESEARCH, Bd.4, Nr.3, 1987 Seiten 255 - 257 L. GU ET AL.

## Beschreibung

Die Erfindung bezieht sich auf einen 2-(1-Pyrrolidinyl)ethyl-Ester der Säure Ketorolac (vgl. z.B. Merck Index, 11. Auflage 1989) oder (±)5-Benzoyl-2,3-dihydro-1H-pyrrolo[1,2-a]pyrrol-1-carbonsäure (vergl. DE-27 31 678 C2) oder (±)-5-Benzoyl-2,3-dihydro-1H-pyrrolizin-1-carbonsäure. Gegenstand der Erfindung sind neben dem oben genannten Ester auch dessen pharmazeutisch akzeptable Additionssalze sowie deren Verwendung für die Zubereitung von oral oder parenteral verabreichbaren Arzneimitteln.

Die nichtsteroiden Analgetika/Antiphlogistika werden gewöhnlich bei der Behandlung von chronischen Entzündungskrankheiten wie Arthritis eingesetzt. Die längere Einnahme solcher Arzneimittel weist jedoch mehrere unerwünschte Nebenwirkungen auf; die wichtigsten davon sind gastrointestinale Reizung und Ulzeration, was ein noch ungelöstes therapeutisches Problem darstellt.

Für die stärksten nichtsteroiden Analgetika/Antiphlogistika wie die Typen, die mit arylacetischen oder arylpropionischen Säuren in Verbindung stehen, wurde festgestellt, daß das Verhältnis therapeutische Wirkung zu unerwünschten Nebenwirkungen bei subkutaner Verabreichung praktisch dasselbe ist wie bei oraler Verabreichung (wobei die letztgenannte für den Patienten viel angenehmer ist). Es wurde davon ausgegangen, daß diese unerwünschten Nebenwirkungen nicht davon abhängen, ob der direkte Kontakt der Substanz mit den Magen-Darm-Zellen in löslich-konzentrierter oder in fester Form stattfindet, sondern vielmehr von den Plasmaniveaus des Wirkstoffes abhängig ist (cf. Lombardino (ed.), Nonsteroidal Antiinflammatory Drugs; Otterness and Bliven, Chapter 4, pp. 229-230). Deshalb wurde behauptet, daß die Ester dieser analgetischen/entzündungshemmenden Carboxylsäuren nicht frei sein dürften von den unerwünschten Nebenwirkungen der dazugehörigen Säuren. In der Tat werden diese Säuren im Handel im allgemeinen in freier Form (z. B. Indometacin, Naproxen, Ketoprofen, Ibuprofen) oder in Form von Salzen (z. B. Diclofenac-Natriumsalz, Ketorolac-Trometamolsalz) verwendet, aber nicht in Form der Ester; und somit deren Anwendung in Form von Estern vorgeschlagen wurde, liegen diese auch durch ein quaternäres Ammoniumsalz vor (z. B. in EP 289.262). Aus der EP-A-0289262 sind bestimmte Ω-quaternäre Ammoniumalkylester und Thioester mit antiinflammatorische Wirkung bekannt. Insbesondere wird ein Ketorolac-2-(1-Pyrrolidinyl-N-methylammonium)ethylesterchlorid erwähnt.

Antiinflammatorische Substanzen wie Ketorolac sind ganz allgemein erwähnt in: DE-A-2731678, WO-91/13609, J.M. Muchovski, et al. in J. Med. Chem. 1985, 28, 1037-1049, Leo Gu und Robert G. Strickley in Pharmaceutical Research, Band 4, No. 3, 1987, 255-257.

Trotz zahlreicher Versuche wurde somit die Notwendigkeit nicht befriedigend gelöst, neue nichtsteroide analgetische/entzündungshemmende Verbindungen aufzufinden, die die sie kennzeichnende hohe therapeutische Wirkung beibehalten, aber den Magen-Darmtrakt weniger reizen und weniger Geschwüre verursachen.

Die Erfindung stellt ein pharmazeutisch verträgliches Säureadditionssalz des 2-(1-Pyrroli-dinyl)ethylesters der (±)-5-Benzoyl-2,3-dihydro-1H-pyrrolizin-1-carbonsäure der Formel (I) bereit;

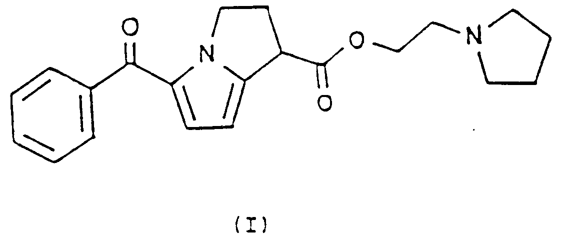

Vorzugsweise handelt es sich hierbei um das Oxalat. Bekanntlich hat das im Handel gebräuchliche Ketorolac-Trometamolsalz unerwünschte Nebenwirkungen, nämlich Magen-Darm-Reizung und Ulzeration. Diese unerwünschten Wirkungen sind bei Anwendung des erfindungsgemäßen Produktes überraschenderweise viel geringer, was dieses zu einem potentiell sehr vorteilhaften Analgetikum/Antiphlogistikum macht, insbesondere für die Behandlung von Schmerzen oder Entzündungen bei Menschen und Tieren.

Gegenstand der Erfindung ist weiterhin die Verwendung des 2-(1-Pyrrolidinyl)ethyl-Esters von Ketorolac oder eines pharmazeutisch verträchlichen Additionssalzes davon zusammen mit pharmazeutisch verträglichen Hilfsstoffen, insbesondere die geeigneten galenischen Formulierungen für orale oder parenterale Verabreichung (Komprimate, Tabletten, Dragees, Kapseln, Pulver, Sirupe, Suspensionen, injizierbare Lösungen etc.) zur Herstellung eines Arzneimittels für die Behandlung von Schmerzen bei Menschen und Tieren.

Gegenstand der vorliegenden Erfindung ist weiterhin ein Verfahren zur Herstellung des 2-(1-Pyrrolidinyl)ethyl-Esters von Ketorolac, das das "Alkylieren" der (±)-5-Benzoyl-2,3-dihydro-1H-pyrrolizin-1-carbonsäure mit 2-(1-Pyrrolidinyl)ethyl-chlorid oder einem gleichwertigen alkylierenden Reagens und das Zugeben der entsprechenden Säure für die Bildung des gewünschten Additionssalzes, das durch Kristallisation isoliert wird, umfaßt.

Wie die Vergleichsversuche gemäß Tabelle 1 zeigen, ist die pharmakologische Wirkung des Oxalsäuresalzes des 2-(1-Pyrrolidinyl)ethyl-Esters des Ketorolac (B in der Tabelle) dem in handelsüblichen Formulierungen verwendeten Ketorolac-Trometamol-Salzes (A in der Tabelle) ähnlich. Das die analgetischen/antiphlogistischen und antipyretischen Wirkungen beider Produkte untereinander ähnlich sind, ist angesichts des Standes der Technik überraschend.

Noch überraschender ist, daß, so wie es die Ergebnisse der Vergleichsversuche in Tabelle 2 belegen, das Oxalsäuresalz des 2-(1-Pyrrolidinyl)ethyl-Esters des Ketorolac (Spalte B) viel geringere unerwünschte Nebenwirkungen hat als das Trometamolsalz von Ketorolac (Spalte A). Somit wird beobachtet, daß der Ulzerationsindex mit (B) lediglich ein Drittel des bei (A) festgestellten ist; daß im Versuch der Verletzungsmöglichkeit des Magengewebes mit (B) alle Tiere unverändert bleiben, während mit (A) alle Hämorrhagien oder Nekrosen aufwiesen;
und daß der Zerstörungsindex der Magenschleimhaut bei (B) ebenfalls sehr viel geringer ist als bei (A). Und dies alles bei einer sowohl für (A) als auch für (B) akzeptablen akuten Toxizität. Der 2-(1-Pyrrolidinyl)ethyl-Ester des Ketorolac erweist sich deshalb als potentiell vorteilhafter bei der Therapie als Ketorolac.

Im Stand der Technik gibt es nichts, daß auf das überraschend niedrige Niveau an unerwünschten Nebenwirkungen hinweist, das der 2-(1-Pyrrolidinyl)ethyl-Ester von Ketorolac aufweist. Dieses Niveau ist zum großen Teil auf das Vorhandensein des Rings von 5 Pyrrolidin-Gliedern zurückzuführen, der entscheidend ist, wie nachstehend noch gezeigt wird.

Wenn anstelle des Pyrrolidin-Ringes dieselben Atome vorhanden sind (mit zwei zusätzlichen Wasserstoffatomen), jedoch in offener Form, ist die pharmakologische Wirkung praktisch dieselbe, aber die Vorteile der geringeren unerwünschten Nebenwirkungen verschwinden. Dies ist in den Spalten (C) von Tabelle 1 und 2 aufgezeigt, wo die Ergebnisse der Vergleichstests für das Maleat des 2-(Diethylamino)ethyl-Esters von Ketorolac angegeben sind. In der Tabelle ist festzustellen, daß der Ulzerationsindex von 2-(Diethylamino)ethyl-Ester doppelt so hoch ist wie bei dem 2-(1-Pyrrolidinyl)ethyl-Ester, und die Gefahr der Magenschleimhautverletzung ist beim ersteren sehr viel größer als beim zweiten. Sie ist praktisch die gleiche wie die des Trometamolsalzes. Etwas Ähnliches geschieht, wenn anstelle von zwei Ethylgruppen zwei Methylgruppen treten, was in den Versuchen (die nicht in der Tabelle aufgeführt sind) mit dem 2-(Dimethylamino)ethyl-Ester von Ketorolac nachgewiesen wurde.

Wenn anstelle eines Pyrrolidin-Ringes (mit fünf Gliedern) der Ring sechs Glieder hat, werden die genannten Vorteile auch nicht erzielt, was in Versuchen (die nicht in der Tabelle aufgeführt sind) mit dem entsprechenden 2-(1-Piperidinyl)ethyl-Ester und mit dem 2-(4-Morpholinyl)ethyl-Ester nachgewiesen wurde.

Die hohe pharmakologische Wirkung und das überraschend niedrige Niveau an unerwünschten Nebenwirkungen des 2-(1-Pyrrolidinyl)-ethyl-Esters von Ketorolac und seinen pharmazeutisch verträglichen Additionssalzen im Vergleich zu Ketorolac und dessen anderen, strukturell ähnlichen Estern (die hier zum ersten Mal zubereitet wurden) machen das erfindungsgemäße Produkt besonders nützlich und vorteilhaft als analgetisches/entzündungshemmendes Mittel.

### Vergleichsversuche zur erwünschten pharmakologischen Wirkung

In Tabelle 1 sind die Vergleichsergebnisse über die analgetische, die antiphlogistische und antipyretische Wirkung angegeben, die bei oraler Gabe des handelsüblichen Trometamolsalzes von Ketorolac (A), von 2-(1-Pyrrolidinyl)ethyl-Ester von Ketorolac (B Gegegenstand der vorliegenden Erfindung) als Oxalat und von 2-(Diethylamino)ethyl-Ester von Ketorolac (C = Referenzzubereitung) bei einer Ratte erzielt wurden.

Die analgetische Wirkung wurde mit dem Test der Inhibition der erzeugten Kontorsionen durch 0.1 ml 3%ige Essigsäure gemessen, die peritoneal verabreicht wurde.

Die entzündungshemmende Wirkung wurde mit der Paw-Ödem-Methode bewertet, wobei 1%iges Karragenin verwendet wurde, wobei der Prozentsatz der Abnahme der Volumenerhöhung an der Pfote der Ratte gemessen wurde.

Die antipyretische Wirkung wurde dadurch bestimmt, daß die Temperatur der Ratte mit Bierhefe auf 20 % erhöht wurde, und die Rektaltemperatur der Ratte 5, 6, 7 und 8 Stunden nach der Gabe gemessen wurde. Es wurde der Prozentsatz der Erhöhung gegenüber dem Vergleichsmuster gemessen.

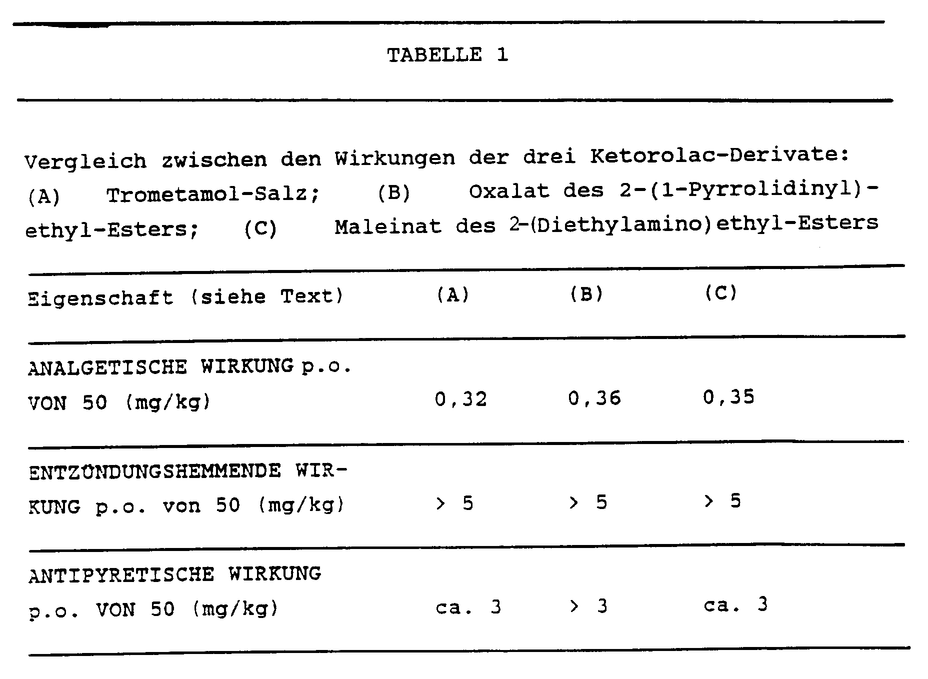

### Vergleichsversuche der unerwünschten Nebenwirkungen

In Tabelle 2 werden die Vergleichsergebnisse für die akute Toxizität, Ulzerationswirkung, Läsionsmöglichkeiten des Magengewebes und Zerstörung der Magenschleimhaut für die orale Gabe von (handelsüblichem) Ketorolac-Trometamol-Salz, dem erfindungsgemäßen Oxalat des 2-(1-Pyrrolidinyl)ethyl-Esters von Ketorolac und des Maleinats des 2-(Diethylamino)ethyl-Esters von Ketorolac (hier als Referenzzubereitung) bei der Ratte gezeigt. Die akute Toxizität ist in LD₅₀ ausgedrückt und ist für die drei Fälle vergleichbar und akzeptabel.

Die Ulzerationswirkung wurde in drei Gruppen von jeweils sechs Ratten bestimmt, wobei die drei vorgenannten Produkte in Dosen von jeweils 62.5, 100 und 100 mg/kg p.o. verabreicht wurden. Die Ulzerationswirkung wurde makroskopisch ausgewertet mittels eines Ulzerationsindexes, der der Summe der Bewertungen eines jeden Tieres entspricht, multipliziert mit dem Prozentsatz der Tiere über null, geteilt durch die Gesamtzahl der Tiere (0 = normal; 1 = kleine Hämorrhagien; 2 große Hämorrhagien; 3 = kleiner Ulcus; 4 = großer Ulcus, mehr als 2 mm; 5 = perforierter Ulcus).

Bei denselben Tieren wurde der Grad der Verletzungsmöglichkeit des Magengewebes mittels einer mikroskopischen Quantifzierung der verschiedenen Läsionsgrade bestimmt: ohne Veränderungen, Hämorrhagie und Nekrose.

Die Zerstörung der Magenschleimhaut wurde nach dem Inhalt des Magenschleims als Zellschutzfaktor bei den mit den Verbindungen A und B behandelten Tieren nachgewiesen, entweder durch einmalige Gabe von 100 mg/kg p.o. oder durch eine tägliche Gabe von 25 mg/kg p.o., die sieben Tage wiederholt wurde. Bei jedem Experiment wurden 3 Grupppen mit jeweils 6 Ratten verwendet, eine Gruppe wurde als Vergleichsgruppe eingesetzt, und die Ergebnisse wurden in Prozent Reduktion gegenüber dieser Gruppe angegeben.

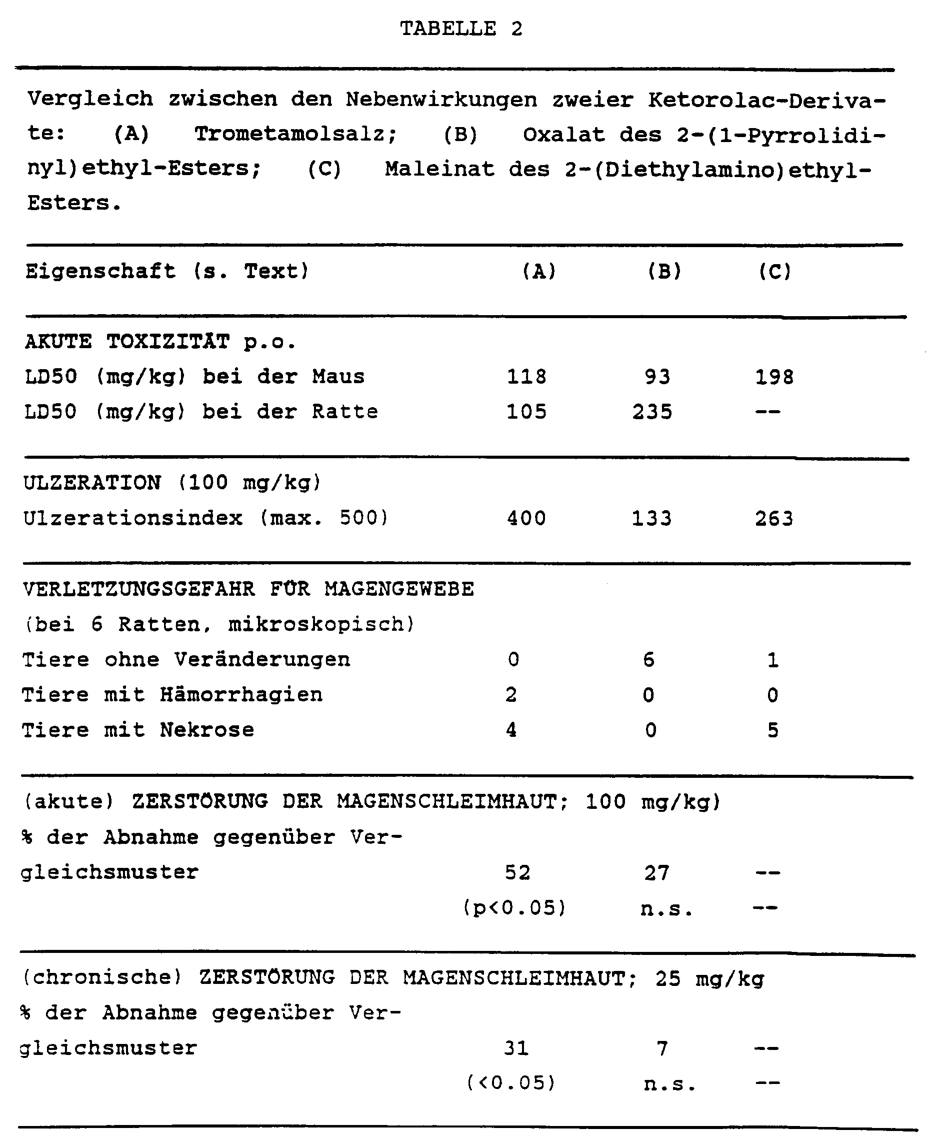

### Herstellungsbeispiel

Zur Herstellung des 2-(1-Pyrrolidinyl)ethyl-Esters des Ketorolac wurde eine Mischung von 2,55 g (10 mmol) (±)-5-Benzoyl-2,3-dihydro-1H-pyrrolizin-1-carbonsäure (Ketorolac) und 4,2 g Natriumcarbonat 30 Minuten lang unter Rückfluß gehalten. Dann wurden 2,00 g (15 mmol) 2-(1-Pyrrolidinyl)ethyl-chlorid-Hydrochlorid hinzugefügt über einen Zeitraum von 15 Stunden unter Rückfluß und kräftigem Rühren. Sie wurde dann konzentriert, indem das Lösungsmittel bei reduziertem Druck entfernt wurde. Der Rückstand wurde in Dichlormethan suspendiert und mehrmals mit einer mit Natriumchlorid gesättigten wässrigen Lösung gewaschen. Die organische Phase wurde über Natriumsulfat getrocknet, gefiltert und das Lösungsmittel durch Vakuumdestillation verdampft. Das Öl wurde in absolutem Ethanol gelöst, und nach und nach wurden 1,26 g wasserfreie Oxalsäure hinzugegeben. Durch Kristallisation wurde das Oxalat des 2-(1-Pyrrolidinyl)-ethyl-Esters des Keterolacs mit einer Ausbeute von 82 % erzielt, der nach der Kristallisation von Ethanol einen Schmelzpunkt von 167-169°C zeigte.

## Patentansprüche

1. Pharmazeutisch verträgliches Säureadditionssalz des 2-(1-Pyrrolidinyl)ethyl-esters der (±)-5-Benzoyl-2,3-dihydro-1H-pyrrolizin-1-carbonsäure der Formel(I)

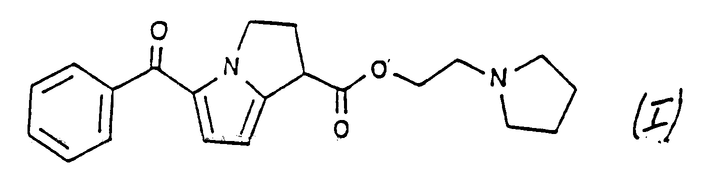

2. Pharmazeutisch verträgliches Säureadditionssalz gemäß Anspruch 1, wobei das genannte Salz Oxalat ist.

3. Verwendung einer Verbindung gemäß den Ansprüchen 1 oder 2 zur Herstellung eines Arzneimittels unter Zusatz von üblichen pharmazeutisch verträglichen Hilfsstoffen zur Behandlung von Schmerzen und Entzündungen bei Menschen und Tieren.

4. Verwendung nach Anspruch 3 zur Herstellung eines Arzneimittels für orale oder parenterale Darbietung.

5. Verfahren zur Herstellung einer pharmazeutisch wirksamen Verbindung gemäß einem der Ansprüche 1 oder 2, dadurch gekennzeichnet, daß (±)-5-Benzoyl-2,3-dihydro-1H-pyrrolizin-1-carbonsäure mit 2-(1-Pyrrolidinyl)ethyl-chlorid oder einem Salz davon oder einem gleichwertigen alkylierenden Reagens alkyliert wird und die entsprechende Säure zur Bildung des gewünschten Salzes zugegeben wird.

## Claims

1. Pharmaceutically acceptable addition salt of 2-(1-pyrrolidinyl)ethylester of (±)-5-benzoyl-2,3-dihydro-1H-pyrrolizine-1-carboxylic acid of formula (I)

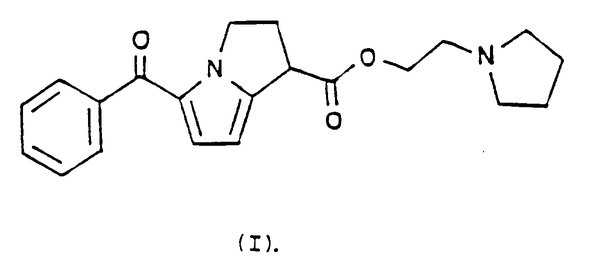

2. A compound according to claim 1 wherein said addition salt is oxalate.

3. Use of a compound according to claim 1 or 2 for the preparation of a medicament including the addition of customary pharmaceutically acceptable excipients for the treatment of pain and inflammations in human beings and animals.

4. The use of claim 3 for the preparation of a medicament for oral or parenteral administration.

5. A process for the preparation of a compound according to claim 1 or 2, characterized in that the (±)-5-benzoyl-2,3-dihydro-1H-pyrrolizine-1-carboxylic acid is alkylated with 2-(1-pyrrolidinyl)-ethylchlorid or a salt thereof or an equivalent alkylating reagent, and the corresponding acid for the formation of the desired addition salt is added.

## Revendications

1. Sel d'addition d'acide pharmaceutiquement tolérable de l'ester 2-(1-pyrrolidinyl) éthylique de l'acide (±)-5-benzoyl-2,3-dihydro-1H-pyrrolizine-1-carboxylique de formule (I)

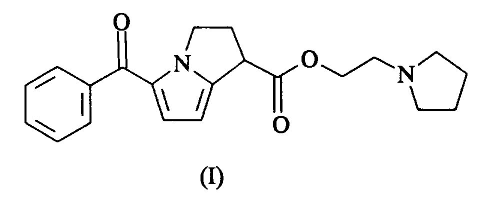

2. Sel d'addition d'acide pharmaceutiquement tolérable selon la revendication 1, ce sel cité étant l'oxalate.

3. Utilisation d'un composé selon les revendications 1 ou 2 pour préparer un médicament obtenu avec addition d'excipients usuels pharmaceutiquement tolérables, pour le traitement de douleurs et d'inflammations des êtres humains et des animaux.

4. Utilisation selon la revendication 3 pour préparer un médicament pour administration par voie orale ou parentérale.

5. Procédé de préparation d'un composé pharmaceutiquement actif selon l'une des revendications 1 ou 2, caractérisé en ce qu'on soumet l'acide (±)-5-benzoyl-2,3-dihydro-1H-pyrrolizine-1-carboxylique à alkylation à l'aide de chlorure de 2-(1-pyrrolidinyl)éthyle ou d'un de ses sels ou d'un réactif équivalent d'alkylation et l'on ajoute l'acide correspondant pour former le sel voulu.
